# EUROPEAN PATENT SPECIFICATION

(11) **EP 3 886 786 B1**
(45) Date of publication and mention of the grant of the patent: **09.12.2026**
(21) Application number: 20814635.7
(22) Date of filing: 09.04.2020
(51) Int. Cl.: A61B 10/04, A61B 10/02

(54) **DEVICE FOR CATCHING A BIOLOGICAL SPECIMEN**
VORRICHTUNG ZUM EINFANGEN EINER BIOLOGISCHEN PROBE
DISPOSITIF DE CAPTURE D'ÉCHANTILLON BIOLOGIQUE

(30) Priority: 29.05.2019 US 201962854010 P
(43) Date of publication of application: 06.10.2021
(73) Proprietor: Atlanta Scientific LLC, Atlanta, GA 30305 (US)
(72) Inventor: ALLEN, Karl, Atlanta, GA 30305 (US); STRANE, Patrick, Atlanta, GA 30309 (US)
(74) Representative: Jacob, Reuben Ellis
(86) International application number: PCT/US2020/027450
(87) International publication number: WO 2020/242607

(56) References cited:
- WO-A1-2004/075740
- WO-A1-2018/088892
- WO-A1-2020/131673
- US-A- 4 376 053
- US-A1- 2006 235 433
- US-A1- 2007 179 341
- US-A1- 2007 191 731
- US-A1- 2014 323 914
- US-A1- 2015 209 491
- US-A1- 2017 160 169
- US-B1- 7 789 246

## Description

### FIELD OF INVENTION

This invention relates to a device and method for catching a biological specimen removed from an individual.

### BACKGROUND

Biological specimens can be captured during surgical procedures such as laparoscopies and endoscopies. During such procedures, medical personnel will extract a biological specimen from an individual and deposit it in a container for transfer to a laboratory or separate room where further testing and study on the specimen can be performed.

Commercially available devices that are placed under suction in order to catch biological specimens typically have vacuum efficiencies below 50%. Lower vacuum performance and efficiency results in wasted energy, lower aspiration forces than expected, and potential challenges during specimen removal. In addition, many prior art devices retain bodily fluid within the device when removing the specimen from the subject. As most specimen catching devices are disposed of once done with a subject, the retained biological waste can build up in the biological waste receptacle, which can lead to contamination and potential leakage when the waste is removed from the waste receptacle.

After extracting a biological specimen using currently available commercial devices, medical personnel must deposit it into a container. Presently, during this transfer it is very likely that the specimen will be touched, accidentally or intentionally, and/or manipulated such that the specimen's integrity is harmed.

There is also a need for a device and method that facilitate the transfer of a biological specimen into a standard specimen container that eliminates the risk of contamination during the transfer. Further, there is an additional need for a device that removes the specimen from bodily fluid of the specimen, eliminating residual. Further, there is a need for a device and method that is minimally disruptive to the medical procedure being performed.

US 2017/0160169 A1 disclosed a specimen collector including a sleeve with one or more interior walls defining a substantially hollow interior cavity, the sleeve further includes an inlet port in fluid communication with the interior cavity and an outlet port in fluid communication with the interior cavity. The sleeve further includes a first side aperture and a second side aperture, the specimen collector also includes a tray slidable within the interior cavity of the sleeve. The tray includes a first specimen well and a second specimen well that alternately may be placed into fluidic communication with the inlet and outlet ports.

WO 2004/075740 A1 disclosed a cut tissue piece recovery device including a tubular body member and a trap member mounted in the body member, where a circulating passage and a trap member mounting port are formed in the body member, a filter part and a head part are formed on the trap member, and the trap part is disposed so that the filter part crosses the circulating passage and the trap member mounting port is closed by the head part when the trap part is mounted in the body member. And the mounting of the trap member in the body member and the removal of the trap member from the body member are freely performed.

WO 2018/088892 A1 disclosed a device for collecting a polyp that has been removed from a body, including a housing with: an inlet connector for coupling an endoscopic tube, an outlet connector for connecting a suction tube, and a collecting space at a position in a flow path between said inlet connector and said outlet connector for containing a polyp collector, said collecting space including a carrying surface for carrying said polyp collector, and the carrying surface of the collecting space at least partially forms part of a movable slide, slidable between a first position where said polyp collector is positioned in said flow path and a second position where said carrying surface is removed from said polyp collector for enabling removal of the polyp collector from the collecting space.

US 4,376,053 disclosed a method for early detection of mastitis in cows, effected during normal milking of cows in a miking parlor, by monitoring the milk taken from each cow for the presence of milk clots, includes cutting the vacuum mile line to each miking station and inserting a filter-detector therein between the claw and the recorder jar. Each filter-detector includes a flat-sided transparent casing with a lateral slot which receives a self-sealing slide carrying the filter element. The side carrier preferably has an integral bypass aperture so that vacuum conditions at the claw end are retained even after the filter element has become blocked, the slider carrier is preferably constructed so that, when in position in the casing, a seepage passage remains between the slide and the casing inner wall, so that cleansing fluid can pass during normal in-place cleaning of the installation.

US 2007/0179341 A1 disclosed an endoscope system, in the endoscope system, a tissue recovery device that recovers tissue is directly mounted on an endoscope, the tissue recovery has a filter portion, and the filter portion is inserted to traverse the conduit of the suction conduit that passes inside the endoscope operation portion. The suction conduit opens to the distal end portion of the endoscope insertion portion, and the working channel branches off from the branch portion that is provided further on the distal end side than the insertion location of the tissue recovery device.

US 2006/235433 A1 discloses features corresponding to the preamble of claim 1.

### SUMMARY

The invention is set out in the appended set of claims.

Further objects, features and advantages will become apparent upon consideration of the following detailed description of the invention when taken in conjunction with the drawings and the appended claims.

### BRIEF DESCRIPTION OF THE DRAWINGS

The following drawings show generally, by way of example, but not by way of limitation, various examples discussed in the present disclosure. In the drawings:
FIG. 1 is a side plan view a device according to an embodiment of the present invention.
FIG. 2 is a front plan view of the device of FIG. 1.
FIG. 3 is a side section view of the device of FIG. 1.
FIG. 3 A is a section detail of FIG. 3 showing the corresponding edges of the removable filter and the main chamber of the device of FIG. 1.
FIG. 4 is a side section view of the device of FIG. 1 showing the main chamber separated from the removable filter.
FIG. 5 is a perspective view of the device of FIG. 1 showing the main chamber separated from the removable filter.
FIG. 6 is a top section view of the device of FIG. 1 showing the main chamber separated from the removable filter.
FIG. 7 is a schematic incorporating the device of FIG. 1 showing how it is connected to other aspects of the present invention during use.
FIG. 8A-8C are graphs showing the performance of the device of FIG. 1 in comparison to commercially available devices.

### DETAILED DESCRIPTION OF THE EMBODIMENTS

Embodiments of the invention will be described more fully hereinafter with reference to the accompanying drawings, in which embodiments of the invention are shown. This invention may be embodied in many different forms and should not be construed as limited to the embodiments set forth herein; rather, these embodiments are provided so that this disclosure will be thorough and complete, and will fully convey the scope of the invention to those skilled in the art.

The present invention is directed to a biological specimen catching device configured for use during medical sampling procedures. In an exemplary aspect, the biological specimen catching device is configured to be utilized with medical procedures that involve suction. The device can capture removed biological specimens, including, but not limited to, polyps. In an aspect, the medical sampling procedures can include colonoscopies, esophagogastroduodenoscopies, and any procedure in which a healthcare provider desires to retrieve a specimen smaller that the aspiration tube.

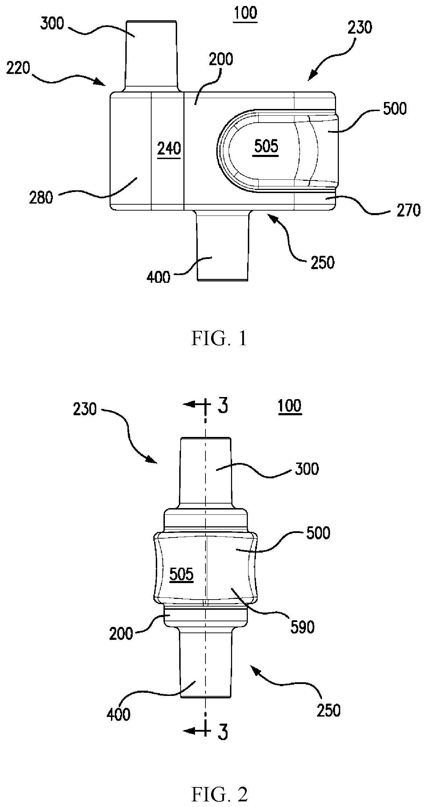

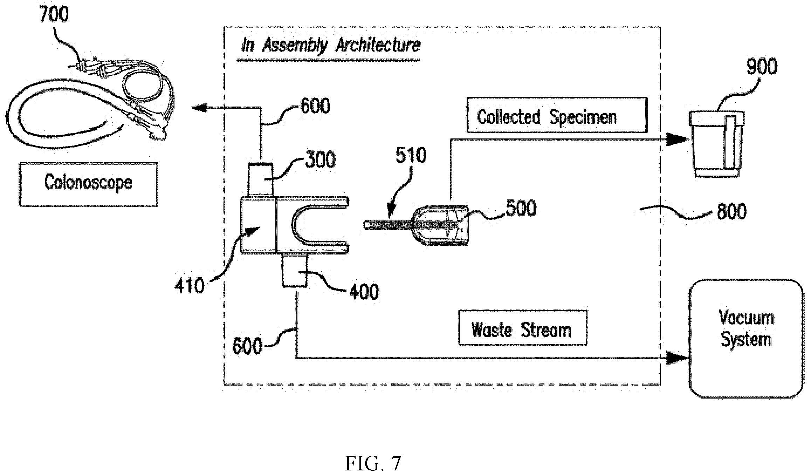

An embodiment of the device 100 is shown in FIGS. 1 - 7. The device 100 comprises a main chamber 200, an inlet 300, an out 400, and a removable filter 500, and is configured to be used with a medical scope 700 and a suction applying device 800. In an aspect, the inlet 300 is configured to be connected to a medical scope 700 and the outlet 400 is configured to be connected to a line 800 of the suction applying device (e.g., a vacuum system) (see FIG. 7). When placed under suction, the device 100 allows for the catching of a biological specimen captured using the working channel of a flexible medical scope 700 such as an endoscope or colonoscope. The device 100 is configured to be compatible with standard endoscopes, colonscopes, and hospital vacuum systems.

FIG. 1 illustrates the device 100 with the removable filter 500 assembled with the main chamber 200. In an aspect, the main chamber 200 comprises an interior portion 210, an exterior portion 220, a top portion 230, a middle portion 240, a bottom portion 250, an aperture 290, an aperture side portion 270, and a non-aperture side portion 280. The side portion 270 of the main chamber 200 and the removable filter 500 are curved, providing superior ergonomics and ensuring ease of manipulation by the end user. Ergonomics/workflow are maximized as the removable filter 500 can be very easily observed and removed/detached from the device 100.

In an aspect, the removable filter 500 is configured for a single use, with multiple removable filters 500 being available for use with the chamber 200. That is, once a specimen has been captured on the removable filter 500, it can be removed and placed into a specimen container, with a new removable filter 500 placed within the chamber 200 for use during the collection process. In addition, new removable filters 500 can be easily and quickly re-inserted into the aperture 290 of the main chamber 200 to minimize the down time between aspirations, as the removal of a filter 500 leads to the loss of aspiration pressure as suction is applied. Additionally, the dimensions of the removable filter 500 allow the medical professional manipulating the biological specimen to minimize the transfer time to the specimen container, with the removable filter 500, while retaining the specimen, is configured to be received within the container.

In an aspect, the main chamber 200 of the device 100 is made from a transparent material, including, but not limited to, clear plastic. In other aspects, other transparent materials can be utilized. While non-transparent materials can be used, it is preferable to use a transparent material in order to see when a biological sample has entered the main chamber 200 and is trapped on the removable filter 500, discussed below. The main chamber 200 is tapered/rounded along its edges between the top portion 230 and bottom portion 250. In an aspect, the top portions 230 and the bottom portions 250 have equal heights to improve the ergonomics of the chamber 200. **In** an exemplary aspect, the main chamber includes a middle portion 240 that is tapered/rounded along the aperture side and non-aperture side portions 270, 280. The tapered nature of the side portions 270, 280 along the middle portion 240 prevents user injury from the exterior surface 220 while reducing the resistance to flow within the interior portion 210 during aspiration and the likelihood of damage to the specimen during extraction/aspiration.

The first and second inlets 300, 400 extend from the top portion 230 and the bottom portion 250 of the chamber 200 respectively, as shown in FIG. 2. **In** an aspect, the inlet 300 and the outlet 400 are tubular extensions in communication with the interior portion 210 of the main chamber 200. The inlets300 and the outlet 400 provide a continuous pathway from the subject via the endoscopic tool 700 and a suction-applying device 800 through the chamber 200 (see FIG. 7). In an aspect, the inlet 300 and the outlet 400 are identical. In an exemplary aspect, the inlet 300 and outlet 400 measure 12 mm vertically with a diameter of 8.39 mm. While the dimensions of the inlet 300 and outlet 400 can vary from those discussed above, these dimensions allow for free airflow during aspiration and ensure compatibility with standard tubing/connectors.

The inlet 300 is oriented on the top portion 230 of the main chamber 200 of the device 100 and the outlet 400 is oriented on the bottom portion 250 of the main chamber 200 of the device 100. **In** an aspect, the inlet 300 and outlet 400 are oriented in a diagonal fashion; i.e., not in direct alignment with one another. This orientation allows for maximal viewing of the collected specimen, as well as for maximum visibility of the removable filter 500. The relative locations of the inlet 300 and outlet 400 also aid in the separation/removal of bodily fluid and bioburden from the biological sample. In an embodiment, the inlet 300 is oriented on the non-aperture side 280 of the main chamber 200 and the outlet 400 is oriented in the middle of the bottom portion 250 of the main chamber 200. While it is possible for the outlet 400 to be arranged closer to the aperture side 270, pooling can occur directly under the filter 500, which makes collection of the specimen more challenging. The alignment of the outlet 400 not directly below the filter 500 and not directly below the inlet 300 minimizes the possibility of fluid pooling within the device 100 and optimizes drainage. In addition, the offset alignment of the inlet 300 and the outlet 400 increase the ergonomics of the device 100, improving the handling of the device 100 by the clinician. In an aspect, the main chamber 200, the inlet 300, and the outlet 400 include an internal taper 310 that prevents the specimen from becoming trapped before reaching the removable filter 500.

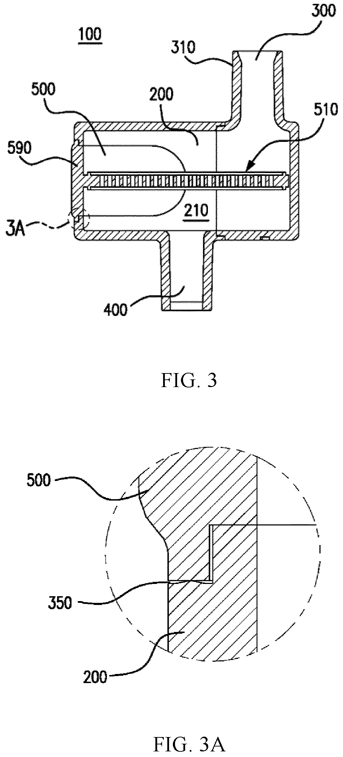

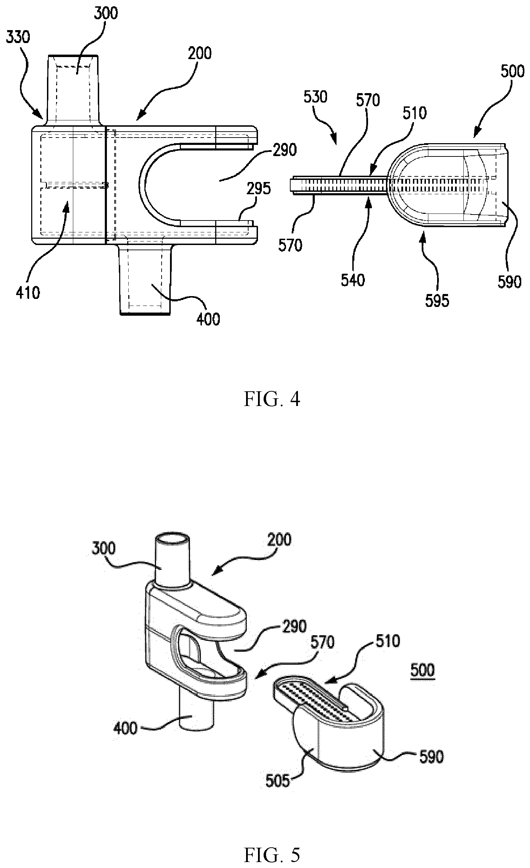

FIG. 3 is a side section view of the device 100 showing the removable filter 500 within the main chamber 200 of the device 100. The removable filter 500 includes a main body 505 and a specimen shelf 510 with a top surface 530 and a bottom surface 540. The specimen shelf 510 includes pores 550 (see FIG. 5). The main body 505 includes a handle portion 590 for the removable filter 500. The edges of the handle portion 590 comprise tapered edges / grooves 595 that intersect with corresponding raised edges / grooves 570 of the specimen shelf 510. In an aspect, the shelf 510 is positioned in the center of the main body 505 of the filter 500. In such aspects, the raised edges/grooves 570 of the specimen shelf 510 are found surrounding the top surface 530 and bottom surface 540. By positioning the shelf 510 in the middle of the main body 505, and also the edges 570 on both sides, the filter 500 can be inserted in two different vertical orientations and still function correctly. That is, the filter 500 is reversible in the vertical orientation, with the filter 500 being able to be inserted with the top surface 530 oriented upwards one way, and the bottom surface 540 being oriented upwards the other way. This increases in the efficiency of replacing the filter 500 as the clinician does not need to be worried about the correct vertical orientation.

FIG. 3 A is a section detail of FIG. 3 showing the corresponding edges of the removable filter 500 and the main chamber 200 of the device. As shown in FIGS. 3-3A, the tapered edges 595 of the handle portion 590 correspond to matching step/grooves 295 found along the aperture 290 of the main chamber 200 that form a lip/groove feature 350 when the removable filter 500 is inserted into the main chamber 200. The combination of the tapered edges 595 with the lip 295 of the aperture form a sealed unit when the removable filter 500 is inserted into the main chamber 200 of the device 100 improving the vacuum efficiency by increasing the quality of the seal between the chamber 200 and the removable filter 500.

Referring to FIGS. 4 - 5, the length and width of the specimen shelf 510, including its curved edges, are configured to match the dimensions of the interior portion 210 of the main chamber 200 along the middle portion 240, to help form a seal. The raised edge 570 of the specimen shelf 510 extends upwards from the top surface 530 and aids in the retention of the specimen on the specimen shelf 510 after the specimen has been removed from the subject, traveled into the inlet 300, traveled into the main chamber 200 and is caught/trapped. In addition, the matching dimensions of the interior 210 and the shelf 510, and the raised edge 570 ensure that the flow of the fluid travels through the pores 550 of the specimen shelf 510, and not around the shelf 510. The handle portion 590 is used to disengage the removable filter 500 from the main chamber 200 of the device 100 before the removable filter is deposited into a specimen container.

FIGS. 4 - 5 show the removable filter 500 separated/disengaged from the main chamber 200 of the device. The removable filter 500 is configured to fit into and be inserted and removed within the aperture 290 in the aperture side portion 270 of the main chamber 200. The main body 505 of the removable filter 500 serves as a handle portion 590 for removing/disengaging the removable filter 500 from the main chamber 200. According to the invention, the handle portion 590 of the removable filter 500 has tapered/curved edges 595 that correspond with the tapered/curved edges 295 of the aperture side portion 270. This provides a seal when the removable filter 500 is inserted into the aperture 290 of the aperture side portion 270 of the main chamber 200 and makes it easy to manipulate the device 100. In an aspect, when the handle portion 590 of the removable filter 500 is compressed, the tapered edges 595 disengage from the corresponding tapered edges 295 of the aperture 290 to easily disengage the removable filter 500 from the main chamber 200.

The removable filter 500 is configured to fit into the main chamber 200 in between the first inlet 300 and the second inlet 400 in between the fluid pathway from the endoscope 700 and the vacuum 800 (see FIG. 7). The removable filter 500 is configured to be inserted and removably retained within the aperture 290 in the side potion 270 of the main chamber 200 creating a seal between the main chamber 200 and the removable filter 500. The biological specimen is extracted from the individual and trapped on the removable filter 500 while the device 100 is placed under suction.

In an aspect, both the main chamber 200 and the removable filter 500 of the device 100 are made from polypropylene material, which provides a better seal between the removable filter 500 and the main chamber 200 of the device 100 when in use. In an aspect, the removable filter 500 is made from plastic or a similar high-contrast material such as ABS, polypropylene and polycarbonate, to allow for easy viewing of the trapped biological specimen within the device 100 and to maximize compatibility with specimen containers 900. In a preferred embodiment, the removable filter 500 is dimensioned to fit entirely within an average sized specimen container 900. In an aspect, such specimen containers 900 can have dimensions that are approximately 45 mm in diameter and 50 mm in height. However, the dimensions can vary.

In an aspect, the main chamber 200 includes a support shelf 410 such that the filter 500 is supported during use whereon the specimen shelf 510 of the removable filter 500 abuts when inserted, ensuring a secure fit and tight seal. In other embodiments, the chamber 200 does not have a shelf 410. The connecting joints 330 (see FIG. 4) between the inlets 300, 400 and the main chamber 200 include small lips to optimize seal performance.

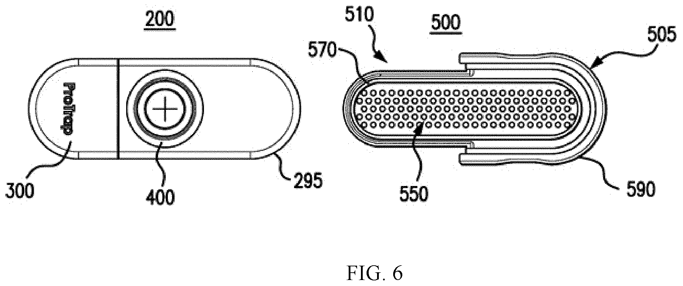

FIG. 6 is a top section view of the device showing the removable filter 500 separated from the main chamber 200 of the device 100. The top surface 530 of the specimen shelf 510 of the removable filter 500 comprises small pores 550 disposed along the top surface 530 of the specimen shelf 510 that allow fluid to pass through when the device 100 is under suction. In an exemplary aspect, each pore 550 has a diameter of approximately 0.75 mm and the pores 550 are spaced approximately 1.3 mm from one another. The pores 550 can take various forms, as well as different geometrical patterns. In an aspect, the pores 550 can have hexagonal, square, or rectangular symmetry. In an aspect, the pores 550 take up approximately 15.5% of the available surface area on the specimen shelf. The spacing of the pores 550 allows the device 100 to maintain high suction when in use while maintaining the appropriate pore size for specimen collection. The spacing and the size of the pores 550 can vary in other embodiments. In other aspects, the pores 550 can take up between 5% to 25% of the surface area of the specimen shelf. Regardless of the different dimensions, the pores 550 should be of a size that allow fluid to travel through and maintain high suction while preventing a specimen from traveling through a pore 550.

FIG. 7 is a schematic representation of the assembly according to an aspect of the present invention. The inlet 300 of the device 100 is connected to a medical scope 700 such as a colonoscope, endoscope or the like via a flexible tube 600. The outlet 400 of the device 100 is connected to a suction line/vacuum system 800 with another flexible tube 600. The flexible tube 600 is made from silicon or like material. The medical scope 700 is inserted into an individual during a surgical procedure such as an endoscopy or laparoscopy. The assembly comprising the medical scope 700 and device 100 is placed under suction using the vacuum system 800. The medical scope 700 is used to isolate/remove the desired biological specimen from the individual. The biological specimen is then suctioned through the first inlet 300, landing on the specimen shelf 510 of the removable filter 500 within the main chamber 200 of the device 100. The removable filter 500 is then detached from the main chamber 200 of the device 100 and the removable filter containing the specimen is deposited into a specimen container 900. The removable filter 500 is replaced with a new/unused removable filter 500 and additional biological specimens can be extracted and deposited as required.

In an exemplary aspect, when the removable filter 500 is inserted into the main chamber 200, this assembly measures 38mm horizontally and 20mm vertically. The dimensions of the device 100 and removable filter 500 allow for the removable filter 500 to be entirely deposited into a specimen container. However, the device 100 and its components are not limited to such dimension, and in other aspects, the dimensions can vary. However, in most aspects, the device 100 and the removable filter 500 have dimensions that allow the removable filter 500 to fit into a specimen container 900 while still maintaining a high suction efficiency when inserted into the main chamber 200.

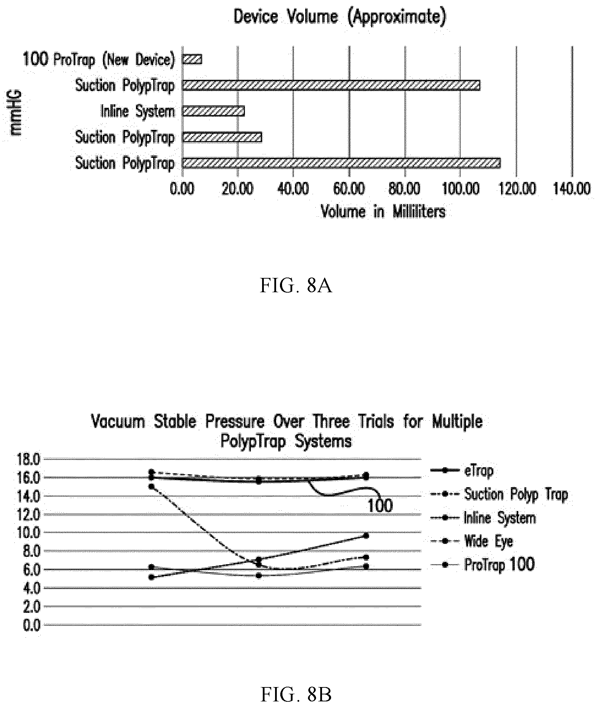

The composition of the device 100 as discussed above has been shown to have benefits over other commercially available products. As shown in FIG. 8 A, the device 100 volume is approximately 400% smaller than the smallest commercially available device on the market. The small, compact size of the device 100 makes the device 100 easy for the user to manipulate use, as well as remove and reinsert removable filters 500 during a medical procedure. The size also ensures that the removable filter 500 and its contents can be deposited entirely into a specimen container.

FIG. 8B shows the vacuum pressure of the device 100 over multiple trials in comparison to commercially available devices. The device 100 exhibits stable pressure over three trials and provides a more consistent performance profile with less variation than other solutions. By employing a closed system featuring optimized seals and ergonomics including the external curvature of the main chamber 200 and removable filter 500, the device 100 is able to maintain stable pressure over multiple uses.

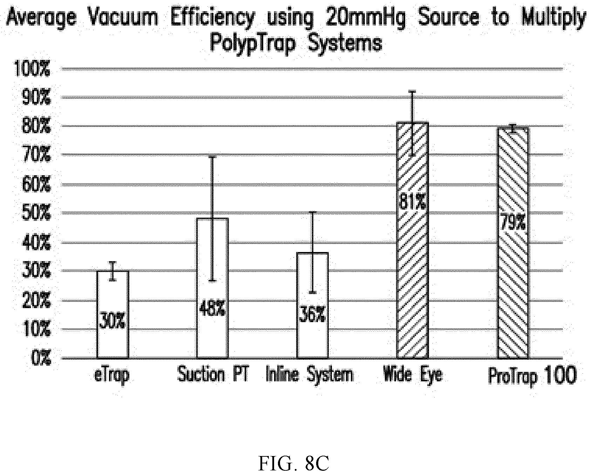

FIG. 8C shows the average vacuum efficiency of the device 100 in comparison to commercially available devices using a 20 inHg/67.7 kPa vacuum source (within range of standard hospital vacuum systems). The device 100 exhibits less variation in its efficiency than any other device. The alternative devices are unable to maintain vacuum efficiency effectively after the being opened/used once and are therefore not ideal for insertion and removal of a removable filter 500 repeatedly during a medical procedure. No commercially available device offers all of the advantages of the present device 100 which include being small enough for easy manipulation, having a removable filter that fits entirely within a specimen container while also maintaining vacuum efficiency and pressure over multiple uses.

Having thus described illustrative embodiments of the present invention, those skilled in the art will appreciate that the disclosures are illustrative only and that various other alternatives, adaptations, and modifications may be made within the scope of the present invention. Accordingly, the present invention is not limited to the specific embodiments as illustrated herein, but is only limited by the following claims.

## Claims

1. A device for catching a biological specimen removed from an individual, the device comprising:
a. a main chamber (200) having an interior portion (210), a top portion (230), a bottom portion (250), and a side portion (270);
b. an inlet (300) connected to a medical scope (700) via a first flexible tube, wherein the inlet (300) is in communication with the interior portion (210) of the main chamber (200) and is oriented on the top portion (230) of the main chamber (200);
c. an outlet (400) connected to a suction line (800) via a second flexible tube, the outlet (400) in communication with the interior portion (210), the outlet (400) oriented on the bottom portion (250) of the main chamber (200), wherein the inlet (300) is positioned opposite the outlet (400); and
d. a removable filter (500) configured to fit into the main chamber (200) and be placed between the inlet (300) and the outlet (400), wherein the removable filter (500) is configured to be inserted and removably retained within an aperture (290) in the side portion (270) of the main chamber (200) creating a seal between the main chamber (200) and the removable filter (500), wherein the specimen is extracted from the individual and trapped on the removable filter (500) while the device is placed under suction;
wherein a main body (505) of the removable filter (500) comprises a handle portion (590) for removing the removable filter (500) from the main chamber (200);
wherein the side portion (270) has a tapered or curved edge (295), and the handle portion (590) is composed of a tapered or curved structure,
**characterized in that** the tapered or curved structure has a tapered or curved edge (595) surrounding the circumference of the tapered or curved structure and corresponding to the tapered or curved edge (295) of the side portion (270), such that when the removable filter (500) is inserted into the aperture (290) of the side portion (270) of the main chamber (200), the tapered or curved edge (595) of the handle portion (590) engages corresponding tapered or curved edge (295) of the side portion (270) of the main chamber (200), thereby the tapered or curved edges (595) of the handle portion (590) forming a seal with the aperture (290).

2. The device according to claim 1, wherein the main chamber (200) is curved at the side portion (270).

3. The device according to claim 1, wherein the main chamber (200) is made from a transparent material.

4. The device according to claim 1, wherein the removable filter (500) further comprises a specimen shelf (510), the specimen shelf (510) extending from the main body (505), wherein the specimen shelf (510) is configured to catch the specimen while within the main chamber (200).

5. The device according to claim 4, wherein the specimen shelf (510) is configured to fully engage inner surfaces of the interior portion (210) of the main chamber (200) so that the specimen lands on the specimen shelf (510).

6. The device according to claim 5, wherein the specimen shelf (510) further comprises pores (550) extending through the specimen shelf (510) to allow fluid to pass through while suction is applied.

7. The device according to claim 5, wherein the specimen shelf (510) further comprises a raised edge (570) extending upwards from a top surface (530) of the specimen shelf (510), the raised edge (570) is configured to retain the specimen when removed from the main chamber (200).

8. The device according to claim 7, wherein the specimen shelf (510) is oriented in the middle of the main body (505) of the filter (500) in a direction from the top surface (530) to a bottom surface (540) of the specimen shelf (510), and wherein the raised edge (570) extends downwards from the bottom surface (540) of the specimen shelf (510), allowing the removeable filter (500) to be inserted into the chamber (200) with either the top surface (530) or the bottom surface (540) oriented in a top position.

9. The device according to claim 1, wherein the removable filter (500) is made from a high-contrast color material.

10. The device according to claim 1, wherein the handle portion (590) is compressible, wherein compressed, the tapered or curved edges (595) disengage from the tapered or curved edges (295) of the aperture (290) to remove the removable filter (500) from the main chamber (200).

11. The device according to claim 1, wherein the removable filter (500) is configured to be removed from the main chamber (200) after capturing the specimen.

12. The device according to claim 1, wherein the inlet (300) and the outlet (400) are oriented diagonally from one another at opposite ends of the device.

13. The device according to claim 1, wherein the inlet (300) is oriented at a corner of the top portion (230) of the device and the outlet (400) is oriented centrally at the bottom portion (250) of the device.

## Patentansprüche

1. Vorrichtung zum Auffangen einer von einer Person entfernten biologischen Probe, wobei die Vorrichtung Folgendes umfasst:
a. eine Hauptkammer (200) mit einem Innenabschnitt (210), einem oberen Abschnitt (230), einem unteren Abschnitt (250) und einem Seitenabschnitt (270);
b. einen Einlass (300), der über einen ersten Schlauch mit einem medizinischen Endoskop (700) verbunden ist, wobei der Einlass (300) mit dem Innenabschnitt (210) der Hauptkammer (200) in Verbindung steht und auf dem oberen Abschnitt (230) der Hauptkammer (200) orientiert ist;
c. einen Auslass (400), der über einen zweiten Schlauch mit einer Saugleitung (800) verbunden ist, wobei der Auslass (400) mit dem Innenabschnitt (210) in Verbindung steht und der Auslass (400) auf dem Bodenabschnitt (250) der Hauptkammer (200) orientiert ist, wobei der Einlass (300) dem Auslass (400) gegenüber positioniert ist; und
d. einen entfernbaren Filter (500), der dazu konfiguriert ist, in die Hauptkammer (200) zu passen und zwischen den Einlass (300) und den Auslass (400) platziert zu werden, wobei der entfernbare Filter (500) dazu konfiguriert ist, in eine Öffnung (290) in dem Seitenabschnitt (270) der Hauptkammer (200) eingesteckt und darin entfernbar gehalten zu werden, wobei eine Dichtung zwischen der Hauptkammer (200) und dem entfernbaren Filter (500) erzeugt wird, wobei die Probe aus der Person entnommen und auf dem entfernbaren Filter (500) gefangen wird, während die Vorrichtung unter Saugwirkung gesetzt wird;
wobei ein Hauptkörper (505) des entfernbaren Filters (500) einen Griffabschnitt (590) zum Entfernen des entfernbaren Filters (500) aus der Hauptkammer (200) umfasst;
wobei der Seitenabschnitt (270) eine sich verjüngende oder gekrümmte Kante (295) aufweist und der Griffabschnitt (590) aus einer sich verjüngenden oder gekrümmten Struktur besteht,
**dadurch gekennzeichnet, dass** die sich verjüngende oder gekrümmte Struktur eine sich verjüngende oder gekrümmte Kante (595) aufweist, die den Umfang der sich verjüngenden oder gekrümmten Struktur umgibt und der sich verjüngenden oder gekrümmten Kante (295) des Seitenabschnitts (270) derart entspricht, dass, wenn der entfernbare Filter (500) in die Öffnung (290) des Seitenabschnitts (270) der Hauptkammer (200) eingesteckt wird, die sich verjüngende oder gekrümmte Kante (595) des Griffabschnitts (590) mit der entsprechenden sich verjüngenden oder gekrümmten Kante (295) des Seitenabschnitts (270) der Hauptkammer (200) in Eingriff kommt, wodurch die sich verjüngenden oder gekrümmten Kanten (595) des Griffabschnitts (590) mit der Öffnung (290) eine Dichtung bilden.

2. Vorrichtung nach Anspruch 1, wobei die Hauptkammer (200) an dem Seitenabschnitt (270) gekrümmt ist.

3. Vorrichtung nach Anspruch 1, wobei die Hauptkammer (200) aus einem durchsichtigen Material hergestellt ist.

4. Vorrichtung nach Anspruch 1, wobei der entfernbare Filter (500) ferner eine Probenablage (510) umfasst und sich die Probenablage (510) von dem Hauptkörper (505) erstreckt, wobei die Probenablage (510) dazu konfiguriert ist, die Probe aufzufangen, während sie sich in der Hauptkammer (200) befindet.

5. Vorrichtung nach Anspruch 4, wobei die Probenablage (510) dazu konfiguriert ist, vollständig mit Innenoberflächen des Innenabschnitts (210) der Hauptkammer (200) in Eingriff zu kommen, damit die Probe auf der Probenablage (510) landet.

6. Vorrichtung nach Anspruch 5, wobei die Probenablage (510) ferner Poren (550) umfasst, die sich durch die Probenablage (510) erstrecken, um Fluid hindurch gelangen zu lassen, während Saugwirkung aufgebracht wird.

7. Vorrichtung nach Anspruch 5, wobei die Probenablage (510) ferner eine erhöhte Kante (570) umfasst, die sich von einer oberen Oberfläche (530) der Probenablage (510) nach oben erstreckt, wobei die erhöhte Kante (570) dazu konfiguriert ist, die Probe zurückzuhalten, wenn sie aus der Hauptkammer (200) entfernt wird.

8. Vorrichtung nach Anspruch 7, wobei die Probenablage (510) in der Mitte des Hauptkörpers (505) des Filters (500) in einer Richtung von der oberen Oberfläche (530) zu einer unteren Oberfläche (540) der Probenablage (510) orientiert ist und wobei sich die erhöhte Kante (570) von der unteren Oberfläche (540) der Probenablage (510) nach unten erstreckt, sodass der entfernbare Filter (500) in die Kammer (200) mit entweder der oberen Oberfläche (530) oder der unteren Oberfläche (540) in einer oberen Lage orientiert eingesteckt werden kann.

9. Vorrichtung nach Anspruch 1, wobei der entfernbare Filter (500) aus einem Material mit kontrastreicher Farbe hergestellt ist.

10. Vorrichtung nach Anspruch 1, wobei der Griffabschnitt (590) zusammendrückbar ist und wenn er zusammengedrückt ist sich die sich verjüngenden oder gekrümmten Kanten (595) von den sich verjüngenden oder gekrümmten Kanten (295) der Öffnung (290) lösen, um den entfernbaren Filter (500) aus der Hauptkammer (200) zu entfernen.

11. Vorrichtung nach Anspruch 1, wobei der entfernbare Filter (500) dazu konfiguriert ist, nach dem Einfangen der Probe aus der Hauptkammer (200) entfernt zu werden.

12. Vorrichtung nach Anspruch 1, wobei der Einlass (300) und der Auslass (400) diagonal zueinander an gegenüberliegenden Enden der Vorrichtung orientiert sind.

13. Vorrichtung nach Anspruch 1, wobei der Einlass (300) an einer Ecke des oberen Abschnitts (230) der Vorrichtung orientiert ist und der Auslass (400) mittig an dem unteren Abschnitt (250) der Vorrichtung orientiert ist.

## Revendications

1. Dispositif servant à capturer un spécimen biologique prélevé sur un individu, le dispositif comportant :
a. une chambre principale (200) ayant une partie intérieure (210), une partie supérieure (230), une partie inférieure (250) et une partie latérale (270) ;
b. une entrée (300) reliée à un endoscope médical (700) par le biais d'un premier tube flexible, dans lequel l'entrée (300) est en communication avec la partie intérieure (210) de la chambre principale (200) et est orientée sur la partie supérieure (230) de la chambre principale (200) ;
c. une sortie (400) reliée à une conduite d'aspiration (800) par le biais d'un deuxième tube flexible, la sortie (400) étant en communication avec la partie intérieure (210), la sortie (400) étant orientée sur la partie inférieure (250) de la chambre principale (200), dans lequel l'entrée (300) est positionnée à l'opposé de la sortie (400) ; et
d. un filtre amovible (500) configuré pour s'adapter dans la chambre principale (200) et pour être placé entre l'entrée (300) et la sortie (400), dans lequel le filtre amovible (500) est configuré pour être inséré et retenu de manière amovible à l'intérieur d'une ouverture (290) dans la partie latérale (270) de la chambre principale (200) pour créer un joint d'étanchéité entre la chambre principale (200) et le filtre amovible (500), dans lequel le spécimen est extrait en provenance de l'individu et emprisonné sur le filtre amovible (500) pendant que le dispositif est placé sous aspiration ;
dans lequel un corps principal (505) du filtre amovible (500) comporte une partie formant poignée (590) servant à retirer le filtre amovible (500) de la chambre principale (200) ;
dans lequel la partie latérale (270) a un bord conique ou incurvé (295), et la partie formant poignée (590) est composée d'une structure conique ou incurvée,
**caractérisé en ce que** la structure conique ou incurvée a un bord conique ou incurvé (595) qui entoure la circonférence de la structure conique ou incurvée et qui correspond au bord conique ou incurvé (295) de la partie latérale (270), de telle sorte que, lorsque le filtre amovible (500) est inséré dans l'ouverture (290) de la partie latérale (270) de la chambre principale (200), le bord conique ou incurvé (595) de la partie formant poignée (590) vient se mettre en prise avec le bord conique ou incurvé correspondant (295) de la partie latérale (270) de la chambre principale (200), les bords coniques ou incurvés (595) de la partie formant poignée (590) formant de ce fait un joint d'étanchéité avec l'ouverture (290).

2. Dispositif selon la revendication 1, dans lequel la chambre principale (200) est incurvée au niveau de la partie latérale (270).

3. Dispositif selon la revendication 1, dans lequel la chambre principale (200) est réalisée à partir d'un matériau transparent.

4. Dispositif selon la revendication 1, dans lequel le filtre amovible (500) comporte par ailleurs une tablette pour spécimen (510), la tablette pour spécimen (510) s'étendant à partir du corps principal (505), dans lequel la tablette pour spécimen (510) est configurée pour capturer le spécimen alors qu'il se trouve à l'intérieur de la chambre principale (200).

5. Dispositif selon la revendication 4, dans lequel la tablette pour spécimen (510) est configurée pour se mettre complètement en prise avec les surfaces intérieures de la partie intérieure (210) de la chambre principale (200) de telle sorte que le spécimen vient se poser sur la tablette pour spécimen (510).

6. Dispositif selon la revendication 5, dans lequel la tablette pour spécimen (510) comporte par ailleurs des pores (550) s'étendant au travers de la tablette pour spécimen (510) pour permettre au fluide de passer au travers tandis que l'aspiration est appliquée.

7. Dispositif selon la revendication 5, dans lequel la tablette pour spécimen (510) comporte par ailleurs un bord relevé (570) s'étendant vers le haut à partir d'une surface supérieure (530) de la tablette pour spécimen (510), le bord relevé (570) est configuré pour retenir le spécimen lorsqu'il est retiré de la chambre principale (200).

8. Dispositif selon la revendication 7, dans lequel la tablette pour spécimen (510) est orientée au milieu du corps principal (505) du filtre (500) dans une direction allant de la surface supérieure (530) à une surface inférieure (540) de la tablette pour spécimen (510), et dans lequel le bord surélevé (570) s'étend vers le bas à partir de la surface inférieure (540) de la tablette pour spécimen (510), ce qui permet au filtre amovible (500) d'être inséré dans la chambre (200) avec soit la surface supérieure (530) soit la surface inférieure (540) orientée dans une position supérieure.

9. Dispositif selon la revendication 1, dans lequel le filtre amovible (500) est réalisé à partir d'un matériau coloré à contraste élevé.

10. Dispositif selon la revendication 1, dans lequel la partie formant poignée (590) est compressible, dans lequel, quand elle est comprimée, les bords coniques ou incurvés (595) se séparent des bords coniques ou incurvés (295) de l'ouverture (290) afin de retirer le filtre amovible (500) de la chambre principale (200).

11. Dispositif selon la revendication 1, dans lequel le filtre amovible (500) est configuré pour être retiré de la chambre principale (200) après la capture du spécimen.

12. Dispositif selon la revendication 1, dans lequel l'entrée (300) et la sortie (400) sont orientées diagonalement l'une par rapport à l'autre à des extrémités opposées du dispositif.

13. Dispositif selon la revendication 1, dans lequel l'entrée (300) est orientée au niveau d'un angle de la partie supérieure (230) du dispositif et la sortie (400) est orientée centralement au niveau de la partie inférieure (250) du dispositif.
